# EUROPEAN PATENT SPECIFICATION

(11) **EP 4 404 986 B1**
(45) Date of publication and mention of the grant of the patent: **23.04.2025**
(21) Application number: 22793394.2
(22) Date of filing: 20.09.2022
(51) Int. Cl.: A61L 27/14, A61L 27/38, A61L 27/60, A61L 27/54

(54) **TWO-LAYER SUPPORT FOR THE PREPARATION OF (EPI)DERMAL EQUIVALENT OR SKIN EQUIVALENT**
ZWEISCHICHTIGER TRÄGER ZUR HERSTELLUNG EINES (EPI)DERMALEN ÄQUIVALENTS ODER HAUTÄQUIVALENTS
SUPPORT BICOUCHE POUR LA PRÉPARATION D'ÉQUIVALENT (ÉPI)DERMIQUE OU D'ÉQUIVALENT DE PEAU

(30) Priority: 20.09.2021 FR 2109858
(43) Date of publication of application: 31.07.2024
(73) Proprietor: L'OREAL, 75008 Paris (FR)
(72) Inventor: RIELLAND, Maïté, 93601 AULNAY-SOUS-BOIS (FR); LYNCH, Barbara, 93601 AULNAY-SOUS-BOIS (FR); GIRARD, Fabien, 93601 AULNAY-SOUS-BOIS (FR); BOYERA, Nathalie, 93601 AULNAY-SOUS-BOIS (FR)
(74) Representative: Lavoix
(86) International application number: PCT/EP2022/076060
(87) International publication number: WO 2023/041798

(56) References cited:
- EVE HEWITT ET AL: "Melt-electrowriting with novel milk protein/PCL biomaterials for skin regeneration", BIOMEDICAL MATERIALS, INSTITUTE OF PHYSICS PUBLISHING, BRISTOL, GB, vol. 14, no. 5, 29 August 2019 (2019-08-29), pages 55013, XP020342852, ISSN: 1748-605X, [retrieved on 20190829], DOI: 10.1088/1748-605X/AB3344
- SILVESTRE BONGIOVANNI ABEL ET AL: "Combination of electrospinning with other techniques for the fabrication of 3D polymeric and composite nanofibrous scaffolds with improved cellular interactions", NANOTECHNOLOGY, INSTITUTE OF PHYSICS PUBLISHING, BRISTOL, GB, vol. 31, no. 17, 11 February 2020 (2020-02-11), pages 172002, XP020352718, ISSN: 0957-4484, [retrieved on 20200211], DOI: 10.1088/1361-6528/AB6AB4

## Description

### TWO-LAYER SUPPORT FOR THE PREPARATION OF (EPI)DERMAL EQUIVALENT OR SKIN EQUIVALENT

EVE HEWITT ET AL: "Melt-electrowriting with novel milk protein/PCL biomaterials for skin regeneration", BIOMEDICAL MATERIALS, INSTITUTE OF PHYSICS PUBLISHING, BRISTOL, GB, vol. 14, no. 5, 29 August 2019, page 55013, discloses a process for skin regeneration using a multilayered biomaterial (1mm thick) obtained by melt-electrowriting" of a milk protein/PCL which displays high porosity seeded with fibroblasts. Structures with large pores are created.

SILVESTRE BONGIOVANNI ABEL ET AL: "Combination of electrospinning with other techniques for the fabrication of 3D polymeric and composite nanofibrous scaffolds with improved cellular interactions", NANOTECHNOLOGY, INSTITUTE OF PHYSICS PUBLISHING, BRISTOL, GB, vol. 31, no. 17, 11 February 2020, page 172002, teaches that the combination of electrospinning with other techniques, in particular melt electrospinning writing (MEW) for the fabrication of 3D polymeric and composite nanofibrous scaffolds improved cell proliferation through the created pores in the nanofibrous assemblies.

The scope of protection is defined by the claims. Any references in the description to methods of treatment refer to the products of the present invention for use in a method for treatment.

The present invention relates to a method for producing a dermal or skin or epidermal equivalent on a two-layer substrate comprising a layer of at least 200 nm in thickness and having a porosity less than or equal to 5 µm formed by electrospinning of a composition comprising at least one polymer, and a layer of at least 20 µm in thickness and having a porosity greater than or equal to 20 µm formed by electrowriting of a composition comprising at least one polymer. The application also relates to the skin or dermal or epidermal equivalent that can be obtained with said method, the use of a dermal equivalent or a skin equivalent or an epidermal equivalent for screening compounds and finally the use thereof in wound dressing or for skin grafts.

Electrospinning (ES) is a method for producing fibers which uses electric force to design yarns charged with polymer solutions or molten polymer. This makes it possible to produce fibers wherein the diameter can go down to 50 nm. However, the deposition is random resulting in a nonwoven fiber mesh. Electrowriting is an emerging technology which uses polymers with controlled deposition of the electrospun fiber and has made it possible to construct complex structures *in situ* or assemble them.

These technologies can be used to produce porous substrates for tissue engineering, especially for reconstructing the dermal and optionally hypodermal part of skin tissues.

At present, lattice models are the most commonly used *in vitro* models for acquiring new knowledge and for evaluation studies. However, they exhibit poor extracellular matrix neosynthesis and poor mechanical properties due to a high water content.

Porous substrates have the advantage of enabling extracellular matrix neosynthesis resulting in a dynamic dermal compartment and are also permissive to numerous cell types. However, for skin tissue reconstruction, porosity is also a limitation as the dermal compartment must be completely filled with matrix to be able to deposit epidermal cells on top and carry out a satisfactory epidermal reconstruction without invaginating keratinocytes in the dermis. This is generally carried out by adding a high concentration of vitamin C to stimulate matrix neosynthesis by the dermal fibroblasts, by selecting specific fibroblasts which secrete a high extracellular matrix content and/or by growing the dermal compartment for a long period of time to enable complete filling. This becomes a real problem when using fibroblasts that are from older donors, and/or exposed to pollution and/or pathological, as they produce less extracellular matrix making it difficult to fill the dermal compartment, which hence makes epidermal reconstruction difficult. The use of vitamin C in the reconstruction of a dermal or skin equivalent is not always desirable for some raw material evaluation studies as it has a relatively broad spectrum of action and can give rise to interferences with the molecules evaluated.

Furthermore, conventional porous substrates have a poor ability to imitate the nanostructural architecture of the skin due to manufacturing limitations.

Electrospinning alone is a solution making it possible to create porous substrates comprising fibers of a diameter close or similar to extracellular matrix fibers. They would help enhance cell adhesion, growth and differentiation. However, due to uncontrolled and low porosity, cell infiltration inside the substrate is complex and mediocre.

The two-layer substrate according to the invention has numerous advantages over known substrates:
- the layer of at least 200 nm in thickness and having a porosity less than or equal to 5 µm is an interface preventing cell passage which makes it possible to reconstruct an epidermis independently of the composition and quantity of the dermal matrix (i.e., it can be reconstructed on an empty or almost empty dermal compartment). It prevents keratinocyte invaginations while allowing the passage of nutrients and proteins as well as communications between the dermis and the epidermis.
- the layer of at least 20 µm in thickness and having a porosity greater than or equal to 20 µm is very porous and therefore enables neosynthesis and remodeling of the extracellular matrix. The design thereof influences cell behavior, extracellular matrix composition and organization, and the mechanical properties of the substrate.

Various two-layer substrates have been created wherein the most porous layer formed has been printed by electrowriting according to various designs. The inventors observed that epidermal reconstruction is possible on these substrates and enables the expression of the main epidermal markers while not being dependent on dermal compartment filling and the design of the most porous layer. Furthermore, the inventors observed that the design of the substrate has an impact on extracellular matrix content and organization and the response to vitamin C.

These substrates also have the advantage of being biocompatible and highly customizable, opening up a new avenue in skin models for new knowledge acquisition, *in vitro* tests, dressing preparation and grafts.

In a first embodiment, the claimed invention relates to a method for producing a dermal equivalent comprising:
a. forming a layer (i) of at least 200 nm in thickness and having a porosity less than 5 µm by electrospinning (ES) or by electrowriting (EW) of a composition comprising at least one polymer,
b. forming a layer (ii) of at least 20 µm in thickness and having a porosity greater than or equal to 20 µm by electrowriting (EW) of a composition comprising at least one polymer,
c. seeding the layer (ii) with dermal and/or hypodermal cells,
wherein step a and step b are carried out sequentially in this order (a followed by b) or in reverse order (b followed by a), and wherein the second electrospinning step takes place longitudinally along the layer created by the first electrospinning step.

The invention further relates to a method for producing a skin equivalent comprising the method for producing a dermal equivalent described above and further comprising after step c) and/or after the two steps a) and b), seeding of the layer (i) with epidermal cells.

In a second embodiment, the claimed invention relates to a method for producing an epidermal equivalent comprising:
a. forming a layer (i) of at least 200 nm in thickness and having a porosity less than or equal to 5 µm by electrospinning (ES) or by electrowriting (EW) of a composition comprising at least one polymer,
b. forming a layer (ii) of at least 20 µm in thickness and having a porosity greater than or equal to 20 µm by electrowriting (EW) of a composition comprising at least one polymer,
c. seeding the layer (i) with epidermal cells,
wherein step a and step b are carried out sequentially in this order (a followed by b) or in reverse order (b followed by a), and wherein the second electrospinning step takes place longitudinally along the layer created by the first electrospinning step.

The claimed invention also relates to a dermal equivalent and a skin equivalent or an epidermal equivalent, that can be obtained with the preparation methods according to the invention.

The claimed invention also relates to the use of a substrate comprising:
a. a layer of at least 200 nm in thickness and having a porosity less than or equal to 5 µm obtained by electrospinning (ES) or by electrowriting (EW) of a composition comprising at least one polymer,
   superimposed longitudinally on
b. a layer of at least 20 µm in thickness and having a porosity greater than or equal to 20 µm, obtained by electrowriting (EW) of a composition comprising at least one polymer,
to form a skin equivalent or a dermal equivalent or an epidermal equivalent.

The claimed invention also relates to the use of a dermal equivalent or a skin equivalent or an epidermal equivalent according to the invention for screening compounds, preferably compounds capable of having a cosmetic, dermatological or pharmaceutical activity for the skin, preferably a cosmetic or dermatological activity after topical application on the skin or injection into the skin.

Another object of the claimed invention further relates to a method for screening a compound having an activity, preferably compounds capable of having a cosmetic, dermatological or pharmaceutical activity for the skin, preferably a cosmetic or dermatological activity after topical application on the skin or injection into the skin, said screening method comprising the application of a candidate compound on the dermal equivalent according to the invention or on the skin equivalent according to the invention or on the epidermal equivalent according to the invention.

The claimed invention also relates to a dermal equivalent according to the invention, a skin equivalent according to the invention, or an epidermal equivalent according to the invention for use thereof in wound dressing or for skin grafts.

### Detailed description of the invention

### Method for producing a dermal equivalent

This invention relates to a method for producing a dermal equivalent comprising:
a. forming a layer (i) of at least 200 nm in thickness and having a porosity less than or equal to 5 µm by electrospinning (ES) or by electrowriting (EW) of a composition comprising at least one polymer,
b. forming a layer (ii) of at least 20 µm in thickness and having a porosity greater than or equal to 20 µm by electrowriting (EW) of a composition comprising at least one polymer,
c. seeding the layer (ii) with dermal and/or hypodermal cells,
wherein step a and step b can be carried out sequentially in this order (a followed by b) or in reverse order (b followed by a), and wherein the second electrospinning step takes place longitudinally along the layer created by the first electrospinning step.

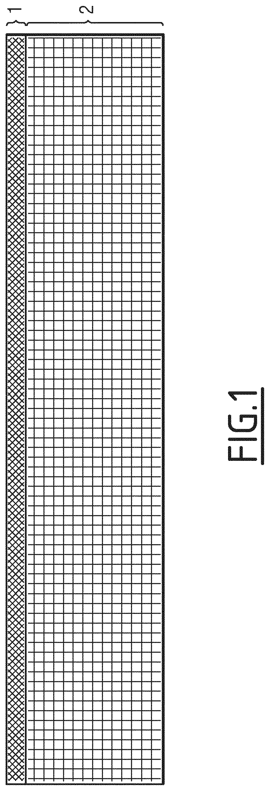

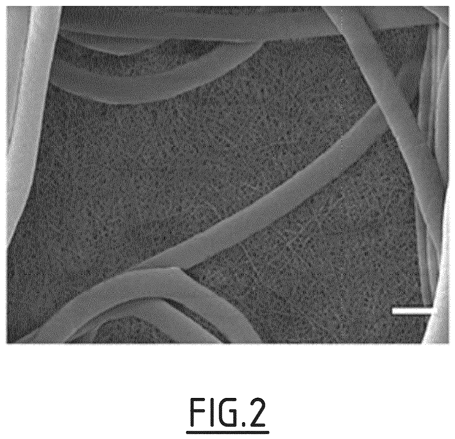

This method therefore makes it possible to obtain a dermal equivalent which is a two-layer substrate comprising dermal and/or hypodermal cells. A schematic representation of this two-layer substrate is shown in figure 1, a photograph of this type of substrate is shown in figure 2.

After steps a and b of the method, a two-layer substrate is obtained. According to a preferred embodiment, the two-layer substrate is a horizontally disposed substantially planar substrate. In this embodiment, the layers are stacked on top of one another.

The layer (i) can be formed first, then the layer (ii) is formed thereon, or conversely the layer (ii) is formed first, then the layer (i) is formed thereon.

The term "electrospinning" (ES) denotes an electrohydrodynamic spraying technique such as solution or melt electrospinning, melt or solution electrowriting, or melt blowing. This technique makes it possible to deposit a polymer fiber.

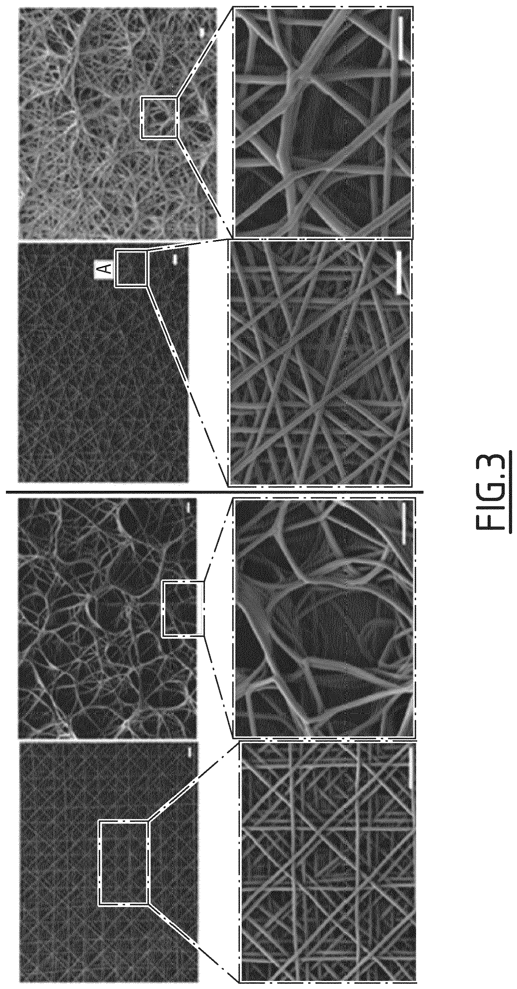

The term "electrowriting" (EW) denotes an electrohydrodynamic spraying technique such as solution or melt electrospinning, melt or solution electrowriting, or melt blowing, wherein the fiber is deposited along a specific outline enable the formation of a predefined design. As an example of a design, mention can be made of octagonal, organic/undulated, or decagonal (see figure 3).

These techniques enable the formation of a layer comprising pores also referred to as porous layer. The term "pore" denotes a hollow space, of varying width and depth, that is optionally closed.

The term "porosity" denotes the ability of a layer to be traversed by elements, in particular via the pores comprised in this layer. Porosity is here defined by the pore size, i.e., the maximum distance measured between the fibers forming a pore. The porosity of a layer can be evaluated with techniques well-known to a person skilled in the art such as scanning electron microscopy and particularly Crossbeam 340 SEM, Zeiss, Oberkochen, Germany.

Thus, "a layer having a porosity less than or equal to 5 µm" denotes a layer comprising pores, and wherein the maximum distance measured between the fibers forming each pore is less than or equal to 5 µm.

Similarly, "a layer having a porosity greater than or equal to 20 µm" denotes a layer comprising pores, and wherein the minimum distance measured between the fibers of each pore is greater than or equal to 20 µm.

Preferably, all the cells seeded on the two-layer substrate are sourced from the same human or animal species, in particular an animal species from the mammal family. The seeded cells can be obtained from healthy donors or from donors suffering from conditions entailing disorders on the dermis and/or skin. These cells can be modified with genetic engineering methods (such as transgenesis for example) particularly to monitor the expression of a gene. Various dermal, skin or epidermal equivalents can be produced in order to study variations associated with various factors such as age, skin type, ethnicity, stress or pollution.

The seeded cells can be obtained from optionally immortal cell cultures, or from primary cultures, or from primary cell strains isolated from skin tissue (without prior culture), preferably all the seeded cell types are obtained from primary cultures.

The term "immortal cells" denotes cells obtained from tumors, spontaneously immortal cells and/or cells immortalized by introducing at least one viral or cellular oncogene. According to a specific embodiment, one or more cell types seeded on the substrate according to the invention are obtained from cultures of cells immortalized by introducing at least one viral or cellular oncogene.

The term "primary culture" denotes a culture of cells obtained directly from an individual's tissue and/or cells. In an alternative embodiment, one or more cell types seeded on the substrate according to the invention are obtained from primary cultures of tissues and/or cells sampled from individuals of the same species and the same age, preferably all the cell types are obtained from primary cultures of tissues and/or cells sampled from individuals of the same species and the same age. According to an embodiment of the invention, one or more and preferably all of the cell types are obtained from adult individuals. In this way, the dermal or skin equivalent according to the invention also makes it possible to study age-related variations or the impact on the dermis or skin of diseases developing during the individual's lifetime. The cells obtained from primary cultures retain contact inhibition unlike immortalized cells, thus the use of these cells makes it possible to limit cell proliferation on the substrate. Furthermore, the use of primary cultures makes it possible to approximate *in vivo* conditions even further.

The term "individual" denotes a subject of a human or animal species, in particular of an animal species of the mammal family.

In a first specific embodiment, the seeded cells are disease model cell types.

In a second specific embodiment, the seeded cells are cells from individuals having a disease, preferably a disease that has been or is suspected of having an influence on the skin.

The term "disease model cell types" denotes cell types from animal or human models reproducing diseases occurring spontaneously or induced by genetic engineering methods (such as transgenesis for example) or with pharmacological tools in order to reproduce the characteristics of cells of individuals suffering from these specific conditions. By way of example, mention can be made of cell types obtained from shFLG (short hairpin filaggrin) models, which lowers filaggrin protein expression, which is in particular a disease model such as atopic dermatitis. Preferably, the diseases according to the invention are diseases which have been or are suspected of having an influence on the skin such as: eczema, lentigo, atopic dermatitis, psoriasis, solar elastosis.

### Step a, formation of a layer (i)

The layer (i) is formed by electrospinning (ES) or by electrowriting (EW) of a composition comprising at least one polymer, and has a thickness of at least 200 nm and a porosity less than or equal to 5 µm.

Preferably, this layer is formed by electrospinning (ES) or by electrowriting (EW) of a composition comprising at least one molten polymer or one polymer in solution, and more preferably a molten polymer.

Preferably, the porosity of the layer (i) is between 0.4 µm and 5 µm. This low porosity prevents cell infiltration but enables the passage of nutrients, growth factors, cytokines, chemokines, etc.

Preferably, the thickness of the layer (i) is between 200 nm and 20 µm, and more preferably between 5 µm and 10µm. This makes it possible to separate the cells while enabling the communication thereof, particularly paracrine communication.

Preferably, the fibers formed by electrospinning of the layer (i) have a diameter between 50 nm and 5 µm, more preferably between 100 nm and 500 nm.

The layer (i) can be substantially flat or comprise reliefs. The term reliefs denotes protuberances and/or hollows. If the layer comprises reliefs, then the latter have a height less than 2 mm, preferably less than 1.5 mm. Said reliefs can be distributed uniformly on the surface of the layer.

### Step b, formation of a layer (ii)

The layer (ii) is formed by electrowriting (EW) of a composition comprising at least one polymer, has a thickness of at least 20 µm and a porosity greater than or equal to 20 µm.

In an embodiment, this layer is formed by electrowriting (EW) of a composition comprising at least one molten polymer or one polymer in solution, and more preferably a molten polymer.

The term "molten polymer" denotes a polymer normally solid at ambient temperature and rendered liquid by increasing the temperature.

The term "polymer in solution" denotes a composition wherein the polymer is dissolved in a solvent.

Thus, for example a 15% polycaprolactone (PCL) solution prepared in a mixed dichloromethane and dimethylformamide solvent is a composition comprising a polymer in solution, whereas PCL pellets preheated to over 60°C, i.e., above the melting point of PCL, form a molten polymer.

Preferably, the thickness of this layer is between 20 µm and 5 mm. This type of thickness making it possible to reproduce the compartments of the dermis and the hypodermis.

Preferably, the porosity of the layer (ii) is between 20 µm and 600 µm. This porosity enables the 3D integration of viable cells, extracellular matrix neosynthesis and organization without applying excessive stress on the cells.

Preferably, the fibers formed by electrospinning of the layer (ii) have a diameter between 50 nm and 100 µm, more specifically between 1 µm and 20 µm. This diameter makes it possible to imitate the diameter of the fibers or fibrils or fiber bundles observed *in vivo* in the extracellular matrix.

The layer (ii) can be substantially flat or comprise reliefs. The term reliefs denotes protuberances and/or hollows. If the layer comprises reliefs, then the latter have a height less than 2mm, preferably less than 1.5mm. Said reliefs can be distributed uniformly on the surface of the layer.

In a first embodiment, the design of this layer can be outlined by substantially rectilinear or curved fibers or a mixture of the two or an overlay of the two. Thus, some fibers can be superimposed or suspended over cavities (see figure 3).

In a second embodiment, the design of this layer can be outlined by different substantially rectilinear fibers in a mixture or superimposed. The term "different" denotes that the orientations thereof are different. Thus, some fibers can be superimposed or suspended over cavities.

In a third embodiment, the design of this layer can be outlined by different curves in a mixture or superimposed. The term "different" denotes that the orientations thereof are different and/or that the curvature thereof is different. Thus, some fibers can be superimposed or suspended over cavities.

In a preferred embodiment of the invention, the layer (ii) has an undulated type or octagonal type or decagonal type design.

### Composition comprising at least one polymer for forming the layers (i) and (ii)

Said at least one polymer of the compositions used to form the layers (i) and (ii) can be synthetic or natural. Said at least one polymer of the compositions used to form the layers (i) and (ii) is preferably bioerodible, bioabsorbable, biocompatible, bioresorbable and/or biodegradable, preferably, it is biocompatible and bioresorbable.

The composition comprising at least one polymer used for forming the layer (i) can be identical to or different from the composition comprising at least one polymer used for forming the layer (ii). Similarly, said at least one polymer used for forming the layer (i) can be identical to or different from that of the composition used for forming the layer (ii).

Examples of suitable polymers comprise, without being limited thereto, poly (alpha-hydroxy acids), polylactide (PLA), polyglycolide (PG), polyethylene glycol (PEG), conjugates of poly (alpha-hydroxy acids), poly(orthoesters) (POE), polyaspirins, polyphosphagens, triethyl-2-acetyl citrate, collagen, peptides of elastin type, starch, pregelatinized starch, chitosans, alginates, albumin, fibrin, silk, homopolymers or copolymers of caprolactone, preferably polycaprolactone, PLCL(poly(lactide-co-caprolactone), poly(2-ethyl-2-oxazine) (PEtOzi), dextrans, vinylpyrrolidone, polyvinyl alcohol (PVA), PVA-g-PLGA, PEGT-PBT copolymer (polyactive), polyethylene oxide - polypropylene oxide - poly(acrylic acid) (PEO-PPO-PAA) copolymer, PLGA-PEO-PLGA copolymer, PEG-PLG copolymer, PLA-PLGA copolymer, poloxamer 407, PEG-PLGA-PEG triblock copolymers, or combinations thereof.

In various embodiments, the composition comprises poly(lactic-co-glycolic acid) copolymer (PLGA), polylactide (PLA), polyglycolide (PGA), D-lactide, homopolymers or copolymers of D,L-lactide, L-lactide, D,L-lactide-co-ε-caprolactone, D,L-lactide-co-glycolide-co-ε-caprolactone, L-lactide-co-ε-caprolactone or poly(ester)amides or a mixture thereof.

Preferably, said at least one polymer of the compositions used to form the layers (i) and (ii) is chosen from:
- natural polymers, for example proteins and polypeptides, glycosaminoglycans, proteoglycans, such as collagen, elastin, hyaluronic acid, dermatan sulfate, gelatin, or mixtures thereof or composites thereof;
- synthetic polymers, for example biodegradable synthetic polymers such as polylactic acid, polyglycolide, poly(lactic-co-glycolic acid) copolymers ("PLGA"), polycaprolactone ("PCL"), poly(dioxanone), poly(trimethylene carbonate) copolymers, polyglyconate, poly(propylene fumarate), poly(ethylene terephthalate), poly(butylene terephthalate), polyethyleneglycol, polycaprolactone copolymers, polyhydroxybutyrate, polyhydroxyvalerate, polycarbonates derived from tyrosine and any random or (multi)block copolymer, or mixtures thereof;
- and mixtures thereof.

Polyethylene glycol methyl ether (mPEG) can be used in the polymer to give it malleability.

In some embodiments, these polymers can also be applied on the fiber formed to provide a sought release profile.

### Step c, seeding of the layer (ii)

The dermal and/or hypodermal cells are preferably seeded at the free surface of the layer (ii). The seeded cells then being able to migrate inside the layer (ii).

The term "dermal and/or hypodermal cells" denotes cells of cellular types present or transiting in the dermis or hypodermis. This comprises but it not limited to fibroblasts, adipocytes, endothelial cells, neuronal cells, hair follicle cells, sweat gland cells, sebaceous gland cells, apocrine gland cells, immune cells (such as macrophages, monocytes, mast cells, lymphocytes, neutrophils, eosinophils, dendritic cells, etc.), nerve cells, vascular smooth muscle cells, specialized muscle cells, stem cells or derived stem cells (such as cells from iPS and ES cells), optionally genetically modified, of human, animal or other origin. In the present application, stem cells are to be understood as excluding human embryonic stem cells.

In a specific embodiment, the seeded dermal and/or hypodermal cells particularly comprise fibroblasts, typically dermal fibroblasts, in particular human fibroblasts, more specifically primary human fibroblasts.

In a preferred embodiment, the seeded fibroblast concentration in the substrate is between 0 and 0.1 million cells/mm³, more preferably from 1500 to 25,000 cells/mm³.

Seeding can be implemented using any suitable technique well-known to a person skilled in the art, and under suitable culture conditions for the growth of dermal and/or hypodermal cells, and in particular fibroblasts.

The term cell seeding denotes controlled or random manual or machine-assisted (using a dispenser or bioprinting) cell deposition.
Once seeded, the dermal and/or hypodermal cells are typically cultured in a suitable culture medium, preferably suitable for fibroblast culture, for example in FHN2D medium (corresponding to DMEM medium supplemented with 2mM of glutamine, antibiotics and 10% fetal calf serum with and without ascorbic acid). They can be cultured submerged or at the air-liquid interface, preferably under suitable conditions for their growth, more specifically preferably at 37°C and at 5% CO₂. Once seeded, the dermal and/or hypodermal cells can be cultured for up to 6 months, preferably from 5 to 40 days.

### Method for producing a skin equivalent

The free surface of the layer (i) can be seeded with epidermal cells, The cell seedings of the layers (i) and (ii) can be carried out simultaneously, or one after the other.

Thus, the invention relates to a method for producing a skin equivalent comprising the method for producing a dermal equivalent according to the invention and further comprising after step c. and/or after the two steps a. and b., seeding of the layer (i) with epidermal cells.

The term "epidermal cells" denotes cells of cellular types present or transiting in the epidermis. This comprises keratinocytes, neuronal cells, melanocytes, Merkel cells, stem cells or derived stem cells (such as cells from iPS and ES cells) and immunocompetent cells, such as Langerhans cells.

The term cell seeding denotes controlled or random manual or machine-assisted (using a dispenser or bioprinting) cell deposition.

In an embodiment, the seeded epidermal cells are keratinocyte type optionally with melanocytes. Preferably, the keratinocytes and the melanocytes are cultured separately before being seeded in or on the substrate.

In a specific embodiment with keratinocytes, the layer (i) can further comprise, by way of bioactive agent in and/or on the fibers, collagen 4 or collagen 7. Advantageously, the thickness of collagen on the layer (i) does not exceed 10 µm.

The epidermal cells are preferably seeded at the free surface of the layer (i).

In a preferred embodiment, the seeded keratinocyte concentration on the substrate is between 0 and 2 million cells/cm², more preferably from 150,000 to 400,000 cells/cm².

Once seeded, the epidermal cells are typically cultured in a suitable culture medium, preferably adapted to the culture of keratinocytes, for example in G7F amplification medium and a G3F differentiation medium (Black et al. (2005) Tissue Eng. 11 :723-733) with or without ascorbic acid. They can be cultured submerged or at the air-liquid interface, for up to 6 months, preferably from 5 to 40 days, at 37°C and at 5% CO₂.

Following an incubation period, preferably of 0 to 7 days, even more preferably of 3 to 7 days, the skin equivalent is preferably maintained at the air/liquid interface, for example by raising the insert, on a metallic grid, or any other cell culture device known to a person skilled in the art enabling the air/liquid interface.

Incubation is then continued, preferably until a skin equivalent displaying the characteristics of a skin is obtained, i.e. a dermal equivalent covered by an epidermal equivalent displaying the four standard types of cell layers, i.e., the basal and suprabasal layers, stratum granulosum and stratum corneum. In this way, incubation is preferably continued for a duration of between 7 and 21 days, even more preferably between 7 and 14 days.

Natural or synthetic compounds can be added on the free or cell-seeded epidermal surface of the layer (i). These compounds are for example water-resistant compounds (for example, a synthetic polymer such as silicone, etc.), UV filters, germicides (as described in Mir et al., Biomatériaux polymères synthétiques pour la cicatrisation: une revue, Progress in Biomaterials (2018), Bibliardi et al., Pansements Bioactifs, Rev. Med. Suisse, 2010 or Tenehaus et al., Agents topiques et pansements pour les soins locaux des brûlures, 2021) or cellulose, alone or in a mixture.

### Method for producing an epidermal equivalent

The invention further relates to a method for producing an epidermal equivalent comprising:
a. forming a layer (i) of at least 200 nm in thickness and having a porosity less than or equal to 5 µm by electrospinning (ES) or by electrowriting (EW) of a composition comprising at least one polymer,
b. forming a layer (ii) of at least 20 µm in thickness and having a porosity greater than or equal to 20 µm by electrowriting (EW) of a composition comprising at least one polymer,
c. seeding the layer (i) with epidermal cells,
wherein step a and step b are carried out sequentially in this order (a followed by b) or in reverse order (b followed by a), and wherein the second electrospinning step takes place longitudinally along the layer created by the first electrospinning step.

The term "epidermal cells" denotes cells of cellular types present or transiting in the epidermis. This comprises keratinocytes, neuronal cells, melanocytes, Merkel cells, stem cells or derived stem cells (such as cells from iPS and ES cells) and immunocompetent cells, such as Langerhans cells.

The term cell seeding denotes controlled or random manual or machine-assisted (using a dispenser or bioprinting) cell deposition.

In an embodiment, the seeded epidermal cells are keratinocyte type optionally with melanocytes. Preferably, the keratinocytes and the melanocytes are cultured separately before being seeded in or on the substrate.

In a specific embodiment with keratinocytes, the layer (i) can further comprise, by way of bioactive agent in and/or on the fibers, collagen 4 or collagen 7. Advantageously, the thickness of collagen on the layer (i) does not exceed 10 µm.

The epidermal cells are preferably seeded at the free surface of the layer (i).

In a preferred embodiment, the seeded keratinocyte concentration on the substrate is between 0 and 2 million cells/cm², more preferably from 150,000 to 400,000 cells/cm².

Once seeded, the epidermal cells are typically cultured in a suitable culture medium, preferably adapted to the culture of keratinocytes, for example in G7F amplification medium and a G3F differentiation medium (Black et al. (2005) Tissue Eng. 11 :723-733) with or without ascorbic acid. They can be cultured submerged or at the air-liquid interface, for up to 6 months, preferably from 5 to 40 days, at 37°C and at 5% CO₂.

Following an incubation period, preferably of 0 to 7 days, even more preferably of 3 to 7 days, the skin equivalent is preferably maintained at the air/liquid interface, for example by raising the insert, on a metallic grid, or any other cell culture device known to a person skilled in the art enabling the air/liquid interface.

Incubation is then continued, preferably until an epidermal equivalent displaying the characteristics sought is obtained, i.e., an epidermal equivalent having the four standard cell layers, i.e. the basal layer, suprabasal layer, stratum granulosum and stratum corneum. In this way, incubation is preferably continued for a duration of between 7 and 21 days, even more preferably between 7 and 14 days.

Optionally, the method can comprise an additional step prior to step c, this step comprising filling or coating of the layer (ii) with one or more bioactive agents, optionally in hydrogel form imitating the dermal matrix.

### In a preferred embodiment, the layer (i) is treated with ethanol prior to step c.

Furthermore, natural or synthetic compounds can be added on the free or cell-seeded epidermal surface of the layer (i). These compounds are for example water-resistant compounds (for example, a synthetic polymer such as silicone, etc.), UV filters, germicides (as described in Mir et al., Biomatériaux polymères synthétiques pour la cicatrisation: une revue, Progress in Biomaterials (2018), Bibliardi et al., Pansements Bioactifs, Rev. Med. Suisse, 2010 or Tenehaus et al., Agents topiques et pansements pour les soins locaux des brûlures, 2021) or cellulose, alone or in a mixture.

### Supplements on the layers (i) and (ii)

In some embodiments of the methods according to the invention, the layer (i) and/or the layer (ii) can furthermore comprise one or more bioactive agent(s) in and/or on the fibers. Bioactive agent or bioactive compound is used here to denote a compound or an entity which modifies, inhibits, activates or affects biological or chemical events. For example, the bioactive agents can include, without being limited thereto, proteins or peptides, preferably of the skin, antibiotics, antiviral substances, enzyme inhibitors, hormones, cell-extracellular matrix interaction modulators, including cell growth inhibitors and anti-adhesion molecules, vasodilatation agents, DNA, RNA or protein synthesis inhibitors, anti-inflammatory agents, anti-angiogenic factors, angiogenic factors, anti-secretory factors, anticoagulants and/or antithrombotic agents, prostaglandins. Preferably, the bioactive agent is different from the polymer forming the composition used for forming the layers (i) and (ii). For example, the bioactive agent can be collagen, gelatin, peptides, ethanol, growth factors, poly-lysine, RGD peptide optionally thiolated, GRGDS peptide (SEQ ID NO: 1) optionally thiolated, as well as the proteins and peptides described in Yamada (1991) J. Biol. Chem. 266 :12809-128012, such as fibronectin or peptides derived from fibronectin such as I and II peptides, laminin or peptides derived from laminin such as the peptides YIGSR (SEQ ID NO: 2), PDSGR (SEQ ID NO: 3), F9, LGTIPG (SEQ ID NO: 4), p20 or PA22-2, vitronectin, fibrinogen, von Willebrand factor, entactin, circumsporozoite protein, thrombospondin or amyloid P component and mixtures thereof. In some embodiments, the bioactive agent can comprise nutraceuticals, such as one or more vitamins for example ascorbic acid, zinc, calcium, or a combination thereof.

The presence of collagen, gelatin, fibronectin, laminin or fibrin typically promotes the adhesion of cells particularly keratinocytes and fibroblasts.

In a specific embodiment with fibroblasts, the collagen used is collagen 1.

In a specific embodiment with keratinocytes, the collagen used is collagen 4 or collagen 7.

In an embodiment, the bioactive agent can be a cell growth promoter such as a sugar, or a combination thereof.

In some embodiments, the bioactive agent is a medicinal product.

The bioactive agents further comprise RNAs, such as siRNAs or shRNAs. In some embodiments, the bioactive agent is a growth factor, a cytokine, an extracellular matrix molecule or a fragment or a derivative thereof, for example, a cell binding site such as the RGD or GRGDS sequence.

The bioactive agent(s) in and/or on the fibers can be deposited on the fibers with different techniques known to a person skilled in the art such as:
- by dipping optionally followed by draining or drying,
- by spraying or nebulization,
- by injection, particularly using a pipette.

Thus, in the methods according to the invention, after steps a and b, the layer (i) can be filled or coated with one or more bioactive agents, optionally in the form of hydrogel imitating the matrix of the dermo-epidermal junction, and/or the layer (ii) can be filled or coated with one or more bioactive agents, optionally in the form of hydrogel imitating the dermal matrix. These steps are preferably carried out before seeding with cells of said layer, if carried out.

The term "filled" denotes that at least 50% of the empty volume of the layer is filled with said active agent(s), preferably at least 70% of the empty volume is filled and more preferably at least 90% of the empty volume is filled. The filling techniques particularly comprise dipping in a bath comprising said bioactive agent(s), injection or deposition of the bioactive agent(s) onto said layer.

The term "coated" or "covered" denotes here that the bioactive agent(s) are deposited on the surface of the fibers forming the layer. The empty spaces of the layer are not filled with said bioactive agent(s). The methods for depositing the bioactive agents and therefore for coating or covering a layer comprise, for example, dipping in a solution comprising the bioactive agent(s) following by a drying or draining step.

The term "hydrogel" denotes a gel wherein the swelling agent is water. The matrix of a hydrogel is generally a polymeric network.

Such hydrogels can comprise for example collagen, gelatin, fibrin, elastin type peptides, agar-Agar, alginate, a decellularized dermal extracellular matrix and mixtures thereof.

By way of example, collagen 4 or 7 or perlecan (glycosamino glycan) is used to imitate the matrix of the dermo-epidermal junction and collagen 1, hyaluronic acid, hyaluronic acid (acrylate of methacrylate), elastin-like peptides (acrylate or methacrylate), gelatin (methacrylate or acrylate), and mixtures thereof are used to imitate the dermal matrix.

In some embodiments of the methods according to the invention, the layer (i) and/or the layer (ii) can be treated and/or modified to increase cell adhesion with any compound or treatment enabling cell adhesion, growth, differentiation, proliferation, migration (such as a chemoattractant of fibroblasts, melanocytes or keratinocytes or other skin cells) of all cell types present or transiting in the skin such as fibroblasts, adipocytes, endothelial cells, neuronal cells, hair follicle cells, sweat gland cells, sebaceous gland cells, apocrine gland cells, sudoral cells, immune cells (such as macrophages, monocytes, mast cells, lymphocytes, neutrophils, eosinophils, dendritic cells, etc.), nerve cells, vascular smooth muscle cells, specialized muscle cells, stem cells or derived stem cells (such as cells from iPS and ES cells), optionally genetically modified, of human, animal or other origin. These treatments comprise for example NaOH treatment, ethanol treatment, plasma treatment after layer formation, or a shish kebab structure during layer formation.

In some embodiments, the fibers of the layer (i) and/or the layer (ii) can be chemically modified, for example with a methacrylate or an acrylate, acrylamide or methacrylamide, which can be crosslinked by covalent bonding after or during fiber formation.

### Equivalents that can be obtained with the methods according to the invention

This invention also relates to a dermal equivalent capable of being obtained with the method according to the invention.

This invention also relates to a skin equivalent that can be obtained with the method according to the invention.

This invention also relates to an epidermal equivalent that can be obtained with the method according to the invention.

### Two-layer substrate

The two-layer substrate can be adapted onto an insert, a nacelle, a suspension system with or without adapter to deposit in on an associated platform (culture plate adapted to inserts (or using an adapter or a supporting element)), or placed on any type of device (cotton wool, grid with legs, etc.) or in a Petri dish if the suspension system has legs or a raising system to enable the air-liquid interface. Commercial examples are: CellCrown^{™} from Sigma, SnapWell^{™} from Corning, NetWell^{™} or TransWell^{™} from Costar, the support plate to be used with Nunc^{™} inserts, or the EPISKIN insert with O-ring (Episkin insert: Episkin nacelle + O-ring (patent FR688226A), see figure 4). The liquid then preferably consists of a suitable culture medium.

Incubation is then continued, preferably until a dermal equivalent, a skin equivalent or an epidermal equivalent is obtained, and preferably until the equivalent has the sought characteristics. For example, for a skin equivalent, incubation is then continued, preferably until an equivalent displaying the characteristics of a skin is obtained, i.e., a dermal equivalent covered by an epidermal equivalent displaying the four standard types of cell layers, i.e., the basal layer, suprabasal layer, stratum granulosum and stratum corneum.

In this way, incubation is preferably continued for a duration of between 7 and 21 days, even more preferably between 7 and 14 days.

### Uses according to the invention

The invention relates to the use of a substrate comprising:
a. a layer of at least 200 nm in thickness and having a porosity less than or equal to 5 µm obtained by electrospinning (ES) or by electrowriting (EW) of a composition comprising at least one polymer,
   superimposed longitudinally on
b. a layer of at least 20 µm in thickness and having a porosity greater than or equal to 20 µm, obtained by electrowriting (EW) of a composition comprising at least one polymer,
to form a skin equivalent or a dermal equivalent or an epidermal equivalent.

Of course, said use is an *in vitro* use.

The layer of at least 20 µm in thickness and having a porosity greater than or equal to 20 µm, can be formed by electrowriting of a composition comprising at least one molten polymer or at least one polymer in solution, preferably the composition comprises at least one molten polymer.

The invention also relates to the use of a dermal equivalent according to the invention or of a skin equivalent according to the invention or of an epidermal equivalent according to the invention for acquiring new knowledge on the dermis or skin or epidermis respectively. Indeed, these equivalents can serve as models in order to study the biology of these tissues, for example in relation to skin aging, effects of ultraviolet radiation, environmental effects, for example, dryness or pollution, or to study diseases.

The invention also relates to the use of a dermal equivalent according to the invention, or of a skin equivalent, or of an epidermal equivalent according to the invention for screening compounds, preferably compounds capable of having a cosmetic, dermatological or pharmaceutical activity for the skin, preferably a cosmetic or dermatological activity after topical application on the skin or injection into the skin.

The term "compounds capable of having a cosmetic, dermatological or pharmaceutical activity for the skin" denotes compounds known to have a cosmetic, dermatological or pharmaceutical activity for the skin, or suspected of having such an activity.

The invention further relates to a method for screening a compound having an activity, preferably compounds capable of having a cosmetic, dermatological or pharmaceutical activity for the skin, preferably a cosmetic or dermatological activity after topical application on the skin or injection into the skin, said screening method comprising the application of a candidate compound on the dermal equivalent according to the invention or on the skin equivalent according to the invention or on the epidermal equivalent according to the invention. Of course, said screening method is an *in vitro* method.

The invention also relates to a dermal equivalent according to the invention or a skin equivalent according to the invention or an epidermal equivalent according to the invention for use thereof in wound dressing or for skin grafts.

The term "wound" denotes a lesion where the skin is grazed, cut, torn, burned or damaged.

Skin grafting is particularly used in cases of burns, diseases such as diabetes, plastic and cosmetic surgery.

For example, in the case of the use of an epidermal equivalent according to the invention in the dressing of a wound, the layer (ii) forms an area conducive to fibroblasts of the treated person. Thus, the person's fibroblasts will be able to migrate and proliferate in this part of the equivalent in order to reconstruct healthy skin at the wound. Furthermore, the layer (ii) of the epidermal equivalent can comprise bioactive agents or agents having undergone treatments to promote fibroblast migration and adhesion.

Similarly, in the case of the use of a dermal equivalent according to the invention in the dressing of a wound, the layer (i) forms an area conducive to keratinocytes of the treated person. Thus, the person's keratinocytes will be able to migrate, proliferate and colonize the layer (i) in order to reconstruct healthy skin at the wound. Furthermore, the layer (i) of the dermal equivalent can comprise bioactive agents or agents having undergone treatments to promote keratinocyte migration and/or proliferation.

The invention also relates to a treatment method comprising the application of a dermal equivalent according to the invention, or of a skin equivalent according to the invention, or of an epidermal equivalent according to the invention. Preferably, the invention relates to a method for treating wounds comprising the administration to an individual in need of a dermal equivalent according to the invention, or of a skin equivalent according to the invention, or of an epidermal equivalent according to the invention.

This invention will be described in more detail in the examples below.

### Figures:

[Fig 1] Figure 1 shows a cross-sectional diagram of the two-layer substrate used for the production of a dermal equivalent, where 1 is the schematic representation of the layer (i) and 2 is the layer (ii).
[Fig 2] Figure 2 shows a photograph of the two-layer substrate used for the production of a dermal equivalent (the bar represents 20 µm). The foreground shows portions of fibers of the layer (ii) and the background shows the less porous layer (i).
[Fig 3] Figure 3 shows different types of design created with electrowriting and tested by the inventors. The scale bar is equivalent to 100 µm. The figure shows, from left to right, an octagonal design with fibers of diameter = 8.7±0.48µm, an undulated design with fibers of diameter = 11.71±0.38µm, a decagonal design with fibers of diameter= 12.38±1.18 µm and an undulated design with fibers of diameter ≈ 14.5µm.

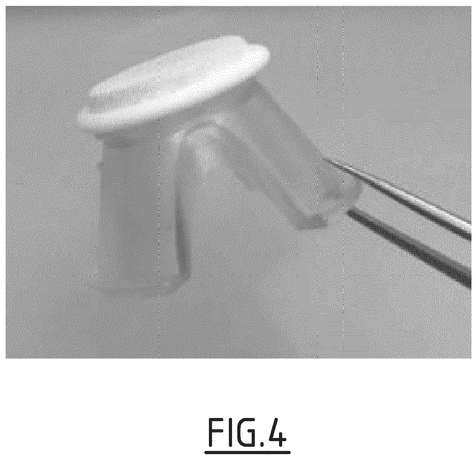

[Fig 4] Figure 4 shows the two-layer substrate mounted on an Episkin insert using an O-ring.

### Examples:

The inventors obtained full-thickness *in vitro* skin models with:
- different designs of the layer (ii) (straight fibers according to an octagonal and decagonal design, and undulated fibers)
- different porosity distributions:
   layer (i): between 0.01 µm² and 0.3 µm²
   straight octagonal melt electrowriting (MEW) layer (ii): between 10 µm² and 500 µm²
   undulated MEW layer (ii): between 20 µm² and 1000 µm²
- different seeded cell concentrations, (fibroblasts: from 58,000 to 1 million cells/cm², keratinocytes from 150,000 to 400,000 cells/cm²)
- different fiber coatings (coating with ethanol, fibronectin, Poly-L-Lysine (PLL)), and plasma treatment
- different fiber diameters (tested from 9 to 14 µm for the layer (ii),
- different culture times (for 11, 18, 28 and 36 days).

Under all the conditions tested, the fibroblasts and keratinocytes were able to bind and reconstruct a dermal (if fibroblasts only) or complete skin model (if fibroblasts and keratinocytes). The keratinocytes form a fully differentiated epidermis with all the layers (basal, stratum spinosum, granulosum and corneum) and express the associated markers (keratin 10 for the stratum spinosum and stratum granulosum and filaggrin for the stratum granulosum and the stratum corneum).

Thanks to the layer (i), the inventors never observed any invagination, infiltration or migration of keratinocytes in the dermis, regardless of the filling of the dermal part. The keratinocytes can be seeded at the same time as the fibroblasts, which is not possible in any porous substrate without a separating membrane between the epidermis and the dermis.

According to the designs, the inventors observed differences. Indeed, extracellular matrix neosynthesis is dependent on the design:
- Elastin is preferentially expressed in the design with straight (and less porous) fibers,
- The collagen 1 content seems to increase in the design with straight fibers.

In general, the organization of the extracellular membrane is correlated with the design.

### Example 1: Two-layer substrate formation with a first undulated design of the layer (ii)

### Materials

Polycaprolactone (PURASORB PC, Corbion Inc., Gorinchem, Netherlands)
Episkin insert: Episkin nacelle and O-ring (described in patent FR688226A)
Solution electrospinning syringes: Henke-Sass, Wolf GmbH; Tuttlingen, Germany
Melt electrowriting syringes: Nordson EFD; Pforzheim, Germany
Scanning electron microscope, TM3030Plus, Hitachi; Tokyo, Japan
Crossbeam 340 scanning electron microscope, Zeiss; Oberkochen, Germany
Melt electrowriting (MEW) printer (Pink), custom; University of (Würzburg or Wuerzburg), Germany
EM ACE600 sputter coating system, Leica; Wetzlar, Germany
Syringe pump, World Precision Instruments; Sarasota, FL, USA
Laser cutting machine, Rayjet ; Plymouth, Michigan, USA

### Electrospinning of the layer (i):

A 15% solution by weight of medical-grade polycaprolactone (PCL) (Corbion, PC-12) was prepared in a mixed dichloromethane and dimethylformamide solvent (DCM:DMF ratio of 3:2). A glass bottle was sealed and the solution was left under stirring overnight. A 5 ml syringe was loaded with the prepared solution and attached to 27G nozzle. A 0.5 ml/h flow rate was used for electrospinning the polymer fibers. A 18 kV voltage difference was applied between the nozzle and the collector. A nozzle-collector distance of 14 cm was used. The electrospun fibers were collected on glass strips mounted on a rotary collector for 30 minutes. The room temperature and humidity were 20.8°C and 43%, respectively.

The fiber-coated glass strips were then used as substrates for the melt electrospinning of the layer (ii).

### Electrowriting of the layer (ii), Undulated design:

A syringe filled with PCL pellets was preheated for at least 24 hours at 75°C. To print sinusoidal grids, a 25G nozzle was used and the nozzle-collector distance was set to 3.75 mm. A nozzle temperature slightly below 70°C was used and a voltage difference of 6kV (+4.5kV at the nozzle and -1.5kV at the collector) was applied to initiate the jet. The pressure used to extrude the polymer was 1.5 bar. A total of 36 layers (12 layers in each direction; three directions) were deposited, where each layer was deposited at an angle of 120° in relation to the previous layer. The wavelength of the sinusoidal waves was set to 2 mm and the amplitude was alternated between 500 µm and 250 µm every three layers. A fiber diameter of 9 to 15 µm approximately, preferably of 10 µm was obtained and the total scaffold height was approximately 400 µm.

### Example 2: Two-layer substrate formation with a second undulated design of the layer (ii)

### Materials

Polycaprolactone (PURASORB PC, Corbion Inc., Gorinchem, Netherlands)
Episkin insert: Episkin nacelle and O-ring (described in patent FR688226A)
Solution electrospinning syringes: Henke-Sass, Wolf GmbH; Tuttlingen, Germany
Melt electrowriting syringes: Nordson EFD; Pforzheim, Germany
Scanning electron microscope, TM3030Plus, Hitachi; Tokyo, Japan
Crossbeam 340 scanning electron microscope, Zeiss; Oberkochen, Germany
Melt electrowriting (MEW) printer (Pink), custom; University of (Würzburg or Wuerzburg), Germany
EM ACE600 sputter coating system, Leica; Wetzlar, Germany
Syringe pump, World Precision Instruments; Sarasota, FL, USA
Laser cutting machine, Rayjet ; Plymouth, Michigan, USA

### Electrospinning of the layer (i):

A 15% solution by weight of medical-grade polycaprolactone (PCL) (Corbion, PC-12) was prepared in a mixed dichloromethane and dimethylformamide solvent (DCM:DMF ratio of 3:2). A glass bottle was sealed and the solution was left under stirring overnight. A 5 ml syringe was loaded with the prepared solution and attached to 27G nozzle. A 0.5 ml/h flow rate was used for electrospinning the polymer fibers. A 18 kV voltage difference was applied between the nozzle and the collector. A nozzle-collector distance of 14 cm was used. The electrospun fibers were collected on glass strips mounted on a rotary collector for 30 minutes. The room temperature and humidity were 20.8°C and 43%, respectively.

The fiber-coated glass strips were then used as substrates for the melt electrospinning of the layer (ii).

### Electrowriting of the layer (ii), Undulated design:

A syringe filled with PCL pellets was preheated for at least 24 hours at 80°C. To print sinusoidal grids, a 25G nozzle was used and the nozzle-collector distance was set to 3.6 mm, with a collector speed of 180mm/min. A nozzle temperature slightly below 80°C was used and a voltage difference of 6kV (+4.5kV at the nozzle and -1.5kV at the collector) was applied to initiate the jet. The pressure used to extrude the polymer was 2 bar. A total of 30 layers (15 layers in each direction; two directions) were deposited, where each layer was deposited at an angle of 90° in relation to the previous layer. A fiber diameter of 13 to 15 µm approximately, preferably of 14 µm was obtained and the total scaffold height was approximately 400 µm.

### Example 3: Two-layer substrate formation with a straight design of the layer (ii)

### Electrospinning of the layer (i)

A 15% solution of medical-grade polycaprolactone (PCL) (Corbion, PC-12) was prepared in a mixed dichloromethane and dimethylformamide solvent (DCM:DMF ratio of 3:2). A glass bottle was sealed and the solution was left under stirring overnight. A 5 ml syringe was loaded with the prepared solution and attached to 27G nozzle. A 0.5 ml/h flow rate was used for polymer fiber electrospinning. A 18 kV voltage difference was applied between the nozzle and the collector. A nozzle-collector distance of 14 cm was used. The electrospun fibers were collected on glass strips mounted on a rotary collector for 30 minutes. The room temperature and humidity were 20.8°C and 43%, respectively.

### Electrowriting of the layer (ii)

### Decagonal design:

A syringe was filled with PCL pellets and preheated for at least 24 hours at 77°C. The loaded syringes were equipped with a 22G nozzle. An air pressure of 1.5 bar was applied to the syringe and a voltage difference of 6 kV (+4.5 kV at the nozzle and -1.5 kV at the collector) was applied to initiate the liquid jet. Stabilization printing was carried out before printing the decagonal structures. The decagonal design is made up of 30 layers of fibers printed in a grid, where each layer has been printed/electrowritten at an angle of rotation of 72° (360/5) with respect to the preceding layer. A fiber spacing of 150 µm was used for each layer. A nozzle-collector distance of 3.6 mm was used for all the prints. A fiber diameter of 8 to 13 µm approximately, preferably of 10 µm was obtained and the total height of the layer produced was approximately 400 µm.

### Example 4: Obtaining a dermal equivalent with two-layer substrate having a straight design of the layer (ii)

To sterilize and increase cell adhesion, the two-layer substrates were treated with ethanol.

To enable culture at the air-liquid interface, the two-layer substrates were mounted on culture inserts with O-rings according to the following positioning: layer (i) inside the insert, layer (ii) at the bottom. The layer (ii) has an octagonal design.

The two-layer substrates were washed with a phosphate buffered saline solution. Then, the two-layer substrates were incubated in a fibroblast culture medium ("FHN2D": DMEM with 2 mM of glutamine, antibiotics and 10% calf serum).

The fibroblasts (NHF) were isolated from skin tissues obtained from plastic surgery after the patient gave their informed consent. They were amplified in an FHN2D medium.

The NHF were trypsinized (trypsin EDTA 0.05%) (4-6min at 37°C), counted and pelleted by centrifugation for 5 min at 190 g.

The pellet was resuspended in a fibroblast medium ("FHN3D": DMEM with 2 mM of glutamine, antibiotics and 10% calf serum and 1 mM ascorbic acid): the final NHF concentration being 6 million NHF/ml of FHN3D medium.

The culture inserts were placed in a Petri dish with the two-layer substrates at the top (layer (ii) on the top).

For NHF seeding: the cell solution was seeded on the inserts on the layer (ii) at a rate of 100 µL/insert i.e., 0.6 million NHF /cm², then, to enable cell adhesion, the inserts with cells were incubated at 37°C for 1 hour.

For the culture step of the dermal equivalent, the inserts were suspended in a 6-well plate with FHN3D medium on top and two-layer substrate at the bottom.

The inserts were then incubated in this FHN3D medium at 37°C, 5% CO₂ for 11 days. The dermal equivalent obtained was observed with an optical microscope and by multiphoton microscopy. The inventors observed the adhesion of the fibroblasts histologically (eosin, hematoxylin, saffron) with a specific fusiform shape of these cells, the filling of the substrate with extracellular matrix (ECM). The multiphoton microscopy showed collagen 1 organization similar to human skin.

### Example 5: Obtaining a dermal equivalent with an undulated design of the layer (ii)

In this example, the same protocol as that of example 3 is applied, except that the two-layer substrate used has a layer with an undulated design for layer (ii).

The dermal equivalent obtained was observed with an optical microscope and by multiphoton microscopy.

The inventors observed that between the dermal equivalent of example 4 and that of example 5, the adhesion of the fibroblasts shown histologically (eosin, hematoxylin and saffron) with a specific fusiform shape of these cells as well as the filling of the substrate with extracellular matrix (ECM) were more homogeneous in example 4 which is potentially due to a heterogeneous porosity in example 5. Moreover, the multiphoton microscopy showed a different collagen 1 fiber organization between examples 4 and 5. These results prove that the change of design induces changes in organization and ECM filling in the substrates.

### Example 6: Obtaining a complete skin equivalent with a straight design of the layer (ii)

In this example, the layer (ii) has a decagonal design. The substrate was prepared similarly to example 4.

In this example, the NHF pellet was resuspended in an FHN3D medium, the final NHF concentration being 4 million NHF/ml of FHN3D medium.

The culture inserts were placed in a Petri dish with the two-layer substrates at the top (layer (ii) on the top). For NHF seeding: the cell solution was seeded on the inserts at a rate of 100µL/insert i.e., 0.4 million NHF /cm², then, to promote cell adhesion, the inserts with cells are incubated at 37°C for 1 hour.

For the culture step of the dermal equivalent, the inserts were suspended in a 6-well plate with FHN3D medium on top and two-layer substrate at the bottom. Then the dermal equivalent was incubated in FHN3D medium at 37°C, 5% CO₂ for 4 days (for 18 days in total of culture) or 14 days (for 36 days in total of culture).

The keratinocytes (NHK) were isolated from skin tissues obtained from plastic surgery after the patient gave their informed consent and then the NHK were amplified in G7F amplification medium (Black et al. (2005) Tissue Eng. 11 :723-733), using feeder cells.

The NHK obtained were trypsinized (trypsin EDTA 0.05% of 8-10 min at 37°C), counted and pelleted by centrifuging for 5 min at 190g, then the pellet was resuspended in G7F amplification medium (with ascorbic acid from 0 to 1mM).

After centrifuging the keratinocytes, they were suspended in G7F medium such that the final NHK concentration is 0.3 million NHK/ml of G7F medium, then they were seeded manually inside the inserts: the final concentration being approximately 0.15million NHK/cm².

The complete skin equivalent was then submerged in a G7F medium with ascorbic acid from 0 to 1mM at 37°C, 5% CO₂ and incubated for 3 days for 18 days in total of culture and 7 days for 36 days in total of culture. Then, the complete skin equivalent was surfaced at the air-liquid interface in a G3F differentiation medium (Black et al. (2005) Tissue Eng. 11 :723-733 + ascorbic acid 0 to 1mM) for 11 for 18 days in total of culture and 15 days for 36 days in total of culture at 37°C, 5% CO₂.

The inventors observed a satisfactory differentiation of the epidermis which has all the expected layers (basal, supra-basal, stratum granulosum and stratum corneum). Moreover, the dermis retains satisfactory fibroblast adhesion and no invagination of the epidermis in the dermis is observed. The synthesis of dermal extracellular matrices (ECM) histologically (eosin, hematoxylin and saffron) and by immunofluorescence such as collagen 1, fibrillin, or elastin, ECM of the dermo-epidermal junction such as Collagen 4 and Perlecan with an increase in the quantity of these proteins between 18 days and 36 days of total culture. Moreover, collagen organization was observed with multiphoton microscopy with winding of the fibers around the fibers of the substrate. An increase in EMC filling was observed in the substrate particularly of collagen 1 (labeled with saffron or observed with multiphoton microscopy) in response to vitamin C.

### Example 7: Obtaining a complete skin equivalent with an undulated straight design of the layer (ii)

In this example, the same protocol as that of example 6 was applied, except that the two-layer substrate used has a layer with an undulated design for layer (ii).

After centrifuging the keratinocytes, they were suspended in G7F medium such that the final NHK concentration is 0.3 million° NHK/ml of G7F medium, then they were seeded manually inside the inserts: the final concentration being approximately 0.15 million NHK/cm².

The complete skin equivalent was then submerged in a G7F medium with ascorbic acid from 0 to 1mM at 37°C, 5% CO₂ and incubated for 7 days. Then, the complete skin equivalent was surfaced at the air-liquid interface in a G3F differentiation medium (Black et al. (2005) Tissue Eng. 11 :723-733) + ascorbic acid 0 to 1mM) for 14 days at 37°C, 5% CO₂.

The inventors observed that between the skin equivalent of example 6 and that of example 7 the synthesis of dermal extracellular matrices (ECM) histologically (eosin, hematoxylin and saffron) and by immunofluorescence such as collagen 1, fibrillin, or elastin, ECM of the dermo-epidermal junction such as Collagen 4 and Perlecan with an increase in the quantity of these proteins between 18 days and 36 days of total culture. Moreover, collagen organization was observed with multiphoton microscopy with winding of the fibers around the fibers of the substrate. An increase in ECM filling in the substrate was observed particularly of collagen 1 (labeled with saffron or observed with multiphoton microscopy) in response to vitamin C. The differences between examples 6 and 7 lie in the more heterogeneous matrix protein organization in example 7 with the presence of empty zones, a lower expression of elastin in example 7 and a different collagen 1 fiber organization (viewed by multiphoton microscopy) with greater collagen 1 fiber bundling in example 7. These results prove that the change of design and porosity gives rise to changes in organization, filling and content of the ECM in the substrates.

## Claims

1. Method for producing a dermal equivalent comprising:
a. forming a layer (i) of at least 200 nm in thickness and having a porosity less than or equal to 5 µm by electrospinning (ES) or by electrowriting (EW) of a composition comprising at least one polymer,
b. forming a layer (ii) of at least 20 µm in thickness and having a porosity greater than or equal to 20 µm by electrowriting (EW) of a composition comprising at least one polymer,
c. seeding the layer (ii) with dermal and/or hypodermal cells,
wherein step a and step b are carried out sequentially in this order or in reverse order, and wherein the second electrospinning step takes place longitudinally along the layer created by the first electrospinning step.

2. Method for producing a skin equivalent comprising the method for producing a dermal equivalent according to claim 1 and further comprising after step c) and/or after the two steps a) and b), seeding of the layer (i) with epidermal cells; and optionally wherein the epidermal cells are cultured submerged or at the air-liquid interface in a suitable medium.

3. Method for producing a dermal equivalent or a skin equivalent according to any one of claims 1 to 2,
- wherein the layer (i) is filled or coated with one or more bioactive agents, optionally in the form of hydrogel imitating the matrix of the dermo-epidermal junction, and/or wherein the layer (ii) is filled or coated with one or more bioactive agents, optionally in the form of a hydrogel imitating the dermal matrix, and/or
- wherein the dermal and/or hypodermal cells are cultured submerged or at the air-liquid interface in a suitable medium.

4. Method for producing an epidermal equivalent comprising:
a. forming a layer (i) of at least 200 nm in thickness and having a porosity less than or equal to 5 µm by electrospinning (ES) or by electrowriting (EW) of a composition comprising at least one polymer,
b. forming a layer (ii) of at least 20 µm in thickness and having a porosity greater than or equal to 20 µm by electrowriting (EW) of a composition comprising at least one polymer,
c. seeding the layer (i) with epidermal cells,
wherein step a and step b are carried out sequentially in this order or in reverse order, and wherein the second electrospinning step takes place longitudinally along the layer created by the first electrospinning step.

5. Production method according to any one of claims 1 to 4, wherein the layer (ii) is formed by electrowriting of a composition comprising at least one molten polymer (melt electrowriting MEW) or comprising at least one polymer in solution.

6. Dermal equivalent that can be obtained with the method according to any one of claims 1, 3 and 5.

7. Skin equivalent that can be obtained with the method according to any one of claims 2 to 3 and 5.

8. Epidermal equivalent that can be obtained with the method according to any one of claims 4 to 5.

9. Use of a substrate comprising:
a. a layer of at least 200 nm in thickness and having a porosity less than or equal to 5 µm obtained by electrospinning (ES) or by electrowriting (EW) of a composition comprising at least one polymer,
superimposed longitudinally on
b. a layer of at least 20 µm in thickness and having a porosity greater than or equal to 20 µm, obtained by electrowriting (EW) of a composition comprising at least one polymer,
to form a skin equivalent or a dermal equivalent or an epidermal equivalent.

10. Use of a substrate according to claim 9, wherein the layer of at least 20 µm in thickness and having a porosity greater than or equal to 20 µm, is formed by electrowriting (EW) of a composition comprising at least one molten polymer (melt electrowriting MEW) or polymer in solution.

11. Use of a dermal equivalent according to claim 6 or of a skin equivalent according to claim 7 or of an epidermal equivalent according to claim 8 for screening compounds, preferably compounds capable of having a cosmetic, dermatological or pharmaceutical activity for the skin, preferably a cosmetic or dermatological activity after topical application on the skin or injection into the skin.

12. Method for screening a compound having an activity, preferably compounds capable of having a cosmetic, dermatological or pharmaceutical activity for the skin, preferably a cosmetic or dermatological activity after topical application on the skin or injection into the skin, said screening method comprising the application of a candidate compound on the dermal equivalent according to claim 6 or on the skin equivalent according to claim 7 or on the epidermal equivalent according to claim 8.

13. Dermal equivalent according to claim 6, for use thereof in wound dressing or for skin grafts.

14. Skin equivalent according to claim 7, for use thereof in wound dressing or for skin grafts.

15. Epidermal equivalent according to claim 8, for use thereof in wound dressing or for skin grafts.

## Patentansprüche

1. Verfahren zum Herstellen eines dermalen Äquivalents, umfassend:
a. Bilden einer Schicht (i) mit einer Stärke von mindestens 200 nm und die eine Porosität von weniger als oder gleich wie 5 µm aufweist, durch Elektrospinnen (ES) oder Elektroschreiben (EW) einer Zusammensetzung, die mindestens ein Polymer umfasst,
b. Bilden einer Schicht (ii) mit einer Stärke von mindestens 20 µm und die eine Porosität von größer als oder gleich wie 20 µm aufweist, durch Elektroschreiben (EW) einer Zusammensetzung, die mindestens ein Polymer umfasst,
c. Bekeimen der Schicht (ii) mit dermalen und/oder hypodermalen Zellen,
wobei Schritt a und Schritt b nacheinander in dieser Reihenfolge oder in umgekehrter Reihenfolge durchgeführt werden, und wobei der zweite Elektrospinnschritt in Längsrichtung entlang der durch den ersten Elektrospinnschritt erzeugten Schicht erfolgt.

2. Verfahren zum Herstellen eines Hautäquivalents, umfassend das Verfahren zum Herstellung eines dermalen Äquivalents nach Anspruch 1 und ferner umfassend nach Schritt c) und/oder nach den zwei Schritten a) und b) ein Bekeimen der Schicht (i) mit epidermalen Zellen; und optional wobei die epidermalen Zellen untergetaucht oder an der Luft-Flüssigkeit-Grenzfläche in einem geeigneten Medium kultiviert werden.

3. Verfahren zum Herstellen eines dermalen Äquivalents oder eines Hautäquivalents nach einem der Ansprüche 1 bis 2,
- wobei die Schicht (i) mit einem oder mehreren bioaktiven Wirkstoffen gefüllt oder beschichtet ist, optional in Form eines Hydrogels, das die Matrix des dermoepidermalen Übergangs imitiert, und/oder wobei die Schicht (ii) mit einem oder mehreren bioaktiven Wirkstoffen gefüllt oder beschichtet ist, optional in Form eines Hydrogels, das die dermale Matrix imitiert, und/oder
- wobei die dermalen und/oder hypodermalen Zellen untergetaucht oder an der Luft-Flüssigkeit-Grenzfläche in einem geeigneten Medium kultiviert werden.

4. Verfahren zum Herstellen eines epidermalen Äquivalents, umfassend:
a. Bilden einer Schicht (i) mit einer Stärke von mindestens 200 nm und die eine Porosität von weniger als oder gleich wie 5 µm aufweist, durch Elektrospinnen (ES) oder Elektroschreiben (EW) einer Zusammensetzung, die mindestens ein Polymer umfasst,
b. Bilden einer Schicht (ii) mit einer Stärke von mindestens 20 µm und die eine Porosität von größer als oder gleich wie 20 µm aufweist, durch Elektroschreiben (EW) einer Zusammensetzung, die mindestens ein Polymer umfasst,
c. Bekeimen der Schicht (ii) mit epidermalen Zellen,
wobei Schritt a und Schritt b nacheinander in dieser Reihenfolge oder in umgekehrter Reihenfolge durchgeführt werden, und wobei der zweite Elektrospinnschritt in Längsrichtung entlang der durch den ersten Elektrospinnschritt erzeugten Schicht erfolgt.

5. Herstellungsverfahren nach einem der Ansprüche 1 bis 4, wobei die Schicht (ii) durch Elektroschreiben einer Zusammensetzung gebildet wird, die mindestens ein geschmolzenes Polymer (Schmelzelektroschreiben MEW) oder mindestens ein Polymer in Lösung umfasst.

6. Dermales Äquivalent, das mit dem Verfahren nach einem der Ansprüche 1, 3 und 5 erlangt werden kann.

7. Hautäquivalent, das mit dem Verfahren nach einem der Ansprüche 2 bis 3 und 5 erlangt werden kann.

8. Epidermales Äquivalent, das mit dem Verfahren nach einem der Ansprüche 4 bis 5 erlangt werden kann.

9. Verwendung eines Substrats, umfassend:
a. eine Schicht mit einer Stärke von mindestens 200 nm und die eine Porosität von weniger als oder gleich wie 5 µm aufweist, die durch Elektrospinnen (ES) oder Elektroschreiben (EW) einer Zusammensetzung erlangt wird, die mindestens ein Polymer umfasst,
das in Längsrichtung überlagert ist auf
b. einer Schicht mit einer Stärke von mindestens 20 µm und die eine Porosität von größer als oder gleich wie 20 µm aufweist, die durch Elektroschreiben (EW) einer Zusammensetzung, die mindestens ein Polymer erlangt wird,
um ein Hautäquivalent oder ein dermales Äquivalent oder ein epidermales Äquivalent zu bilden.

10. Verwendung eines Substrats nach Anspruch 9, wobei die Schicht mit einer Stärke von mindestens 20 µm und die eine Porosität von mehr als oder gleich wie 20 µm aufweist, durch Elektroschreiben (EW) einer Zusammensetzung gebildet wird, die mindestens ein geschmolzenes Polymer (Schmelz-Elektroschreiben MEW) oder ein Polymer in Lösung umfasst.

11. Verwendung eines dermalen Äquivalents nach Anspruch 6 oder eines Hautäquivalents nach Anspruch 7 oder eines epidermalen Äquivalents nach Anspruch 8 zum Screening von Verbindungen, vorzugsweise von Verbindungen, die in der Lage sind, eine kosmetische, dermatologische oder pharmazeutische Wirkung auf die Haut aufzuweisen, vorzugsweise eine kosmetische oder dermatologische Wirkung nach topischer Anwendung auf der Haut oder Injektion in die Haut.

12. Verfahren zum Screening einer Verbindung, die eine Aktivität aufweist, vorzugsweise Verbindungen, die in der Lage sind, eine kosmetische, dermatologische oder pharmazeutische Aktivität für die Haut aufzuweisen, vorzugsweise eine kosmetische oder dermatologische Aktivität nach topischer Anwendung auf der Haut oder Injektion in die Haut, das Screening-Verfahren umfassend ein Auftragen einer Kandidatenverbindung auf den dermalen Äquivalent nach Anspruch 6 oder auf den Hautäquivalent nach Anspruch 7 oder auf den epidermalen Äquivalent nach Anspruch 8.

13. Dermales Äquivalent nach Anspruch 6 zur Verwendung in Wundverbänden oder für Hauttransplantationen.

14. Hautäquivalent nach Anspruch 7 zur Verwendung in Wundverbänden oder für Hauttransplantationen.

15. Epidermales Äquivalent nach Anspruch 8 zur Verwendung in Wundverbänden oder für Hauttransplantationen.

## Revendications

1. Procédé de production d'un équivalent dermique comprenant :
a. la formation d'une couche (i) d'une épaisseur d'au moins 200 nm et d'une porosité inférieure ou égale à 5 µm par électrofilage (ES) ou par électrorécriture (EW) d'une composition comprenant au moins un polymère,
b. la formation d'une couche (ii) d'une épaisseur d'au moins 20 µm et d'une porosité supérieure ou égale à 20 µm par électrorécriture (EW) d'une composition comprenant au moins un polymère,
c. l'ensemencement de la couche (ii) avec des cellules dermiques et/ou hypodermiques,
dans lequel les étapes a et b sont effectuées séquentiellement dans cet ordre ou dans l'ordre inverse, et dans lequel la deuxième étape d'électrofilage se déroule longitudinalement le long de la couche créée par la première étape d'électrofilage.

2. Procédé de production d'un équivalent cutané comprenant le procédé de production d'un équivalent dermique selon la revendication 1 et comprenant en outre, après l'étape c) et/ou après les deux étapes a) et b), l'ensemencement de la couche (i) avec des cellules épidermiques ; et éventuellement dans lequel les cellules épidermiques sont cultivées en immersion ou à l'interface air-liquide dans un milieu approprié.

3. Procédé de fabrication d'un équivalent dermique ou d'un équivalent cutané selon l'une quelconque des revendications 1 à 2,
- dans lequel la couche (i) est remplie ou recouverte d'un ou plusieurs agents bioactifs, éventuellement sous forme d'hydrogel imitant la matrice de la jonction dermo-épidermique, et/ou dans laquelle la couche (ii) est remplie ou recouverte d'un ou plusieurs agents bioactifs, éventuellement sous forme d'hydrogel imitant la matrice dermique, et/ou
- dans lequel les cellules dermiques et/ou hypodermiques sont cultivées en immersion ou à l'interface air-liquide dans un milieu approprié.

4. Procédé de production d'un équivalent épidermique comprenant :
a. la formation d'une couche (i) d'une épaisseur d'au moins 200 nm et d'une porosité inférieure ou égale à 5 µm par électrofilage (ES) ou par électrorécriture (EW) d'une composition comprenant au moins un polymère,
b. la formation d'une couche (ii) d'une épaisseur d'au moins 20 µm et d'une porosité supérieure ou égale à 20 µm par électrorécriture (EW) d'une composition comprenant au moins un polymère,
c. l'ensemencement de la couche (i) avec des cellules épidermiques,,
dans lequel les étapes a et b sont effectuées séquentiellement dans cet ordre ou dans l'ordre inverse, et dans lequel la deuxième étape d'électrofilage se déroule longitudinalement le long de la couche créée par la première étape d'électrofilage.

5. Procédé de fabrication selon l'une quelconque des revendications 1 à 4, dans lequel la couche (ii) est formée par électrorécriture d'une composition comprenant au moins un polymère fondu (melt electrowriting MEW) ou comprenant au moins un polymère en solution.

6. Équivalent dermique pouvant être obtenu par le procédé selon l'une quelconque des revendications 1, 3 et 5.

7. Équivalent cutané pouvant être obtenu par le procédé selon l'une quelconque des revendications 2 à 3 et 5.

8. Équivalent épidermique pouvant être obtenu par le procédé selon l'une quelconque des revendications 4 à 5.

9. Utilisation d'un substrat comprenant :
a. une couche d'une épaisseur d'au moins 200 nm et d'une porosité inférieure ou égale à 5 µm obtenue par électrofilage (ES) ou par électrorécriture (EW) d'une composition comprenant au moins un polymère,
superposée longitudinalement sur
b. une couche d'une épaisseur d'au moins 20 µm et d'une porosité supérieure ou égale à 20 µm, obtenue par électrorécriture (EW) d'une composition comprenant au moins un polymère,
pour former un équivalent cutané ou un équivalent dermique ou un équivalent épidermique.

10. Utilisation d'un substrat selon la revendication 9, dans lequel la couche d'au moins 20 µm d'épaisseur et d'une porosité supérieure ou égale à 20 µm, est formée par électrorécriture (EW) d'une composition comprenant au moins un polymère fondu (melt electrowriting MEW) ou un polymère en solution.

11. Utilisation d'un équivalent dermique selon la revendication 6 ou d'un équivalent cutané selon la revendication 7 ou d'un équivalent épidermique selon la revendication 8 pour cribler des composés, de préférence des composés capables de présenter une activité cosmétique, dermatologique ou pharmaceutique pour la peau, de préférence une activité cosmétique ou dermatologique après application topique sur la peau ou injection dans la peau.

12. Procédé de criblage d'un composé présentant une activité, de préférence des composés capables de présenter une activité cosmétique, dermatologique ou pharmaceutique pour la peau, de préférence une activité cosmétique ou dermatologique après application topique sur la peau ou injection dans la peau, ledit procédé de criblage comprenant l'application d'un composé candidat sur l'équivalent dermique selon la revendication 6 ou sur l'équivalent cutané selon la revendication 7 ou sur l'équivalent épidermique selon la revendication 8.

13. Équivalent dermique selon la revendication 6, destiné à être utilisé pour le pansement des plaies ou pour les greffes de peau.

14. Équivalent cutané selon la revendication 7, destiné à être utilisé pour le pansement des plaies ou pour les greffes de peau.

15. Équivalent épidermique selon la revendication 8, destiné à être utilisé pour le pansement des plaies ou pour les greffes de peau.
